Europäisches Patentamt

European Patent Office

Office européen des brevets

(19)

(11) Numéro de publication: **0 161 123**
**A1**

(12) # DEMANDE DE BREVET EUROPEEN

(21) Numéro de dépôt: **85400447.0**

(22) Date de dépôt: **08.03.85**

(51) Int. Cl.⁴: **A 01 H 15/00**

(30) Priorité: **23.03.84 FR 8404549**

(43) Date de publication de la demande:
**13.11.85 Bulletin 85/46**

(84) Etats contractants désignés:
**AT BE CH DE GB IT LI LU NL SE**

(71) Demandeur: **SOMYCEL**
**Z.I. Route de Tours**
**F-37130 Langeais(FR)**

(72) Inventeur: **Miller, Liliane**
**4, Jardin Ockeghem**
**F-37000 Tours(FR)**

(74) Mandataire: **Laget, Jean-Loup et al,**
**Cabinet Pierre Loyer 18, Rue de Mogador**
**F-75009 Paris(FR)**

(54) Nouveaux pleurotes.

(57) Pleurotes présentant le caractère asporulé, des lamelles décurrentes sur la majeure partie due pied et des lamelles plus développées que celles des autres souches.

EP 0 161 123 A1

- 1 -

## Nouveaux pleurotes

L'invention concerne de nouveaux pleurotes éliminant définitivement les nuisances pour le personnel de culture provoquées par la présence de spores.

Les pleurotes sont en effet des champignons comestibles bien connus, remarquables notamment par la très grande quantité de spores qu'ils libèrent. Ces spores sont des allergènes pour l'homme. De plus, les spores sont des vecteurs qui peuvent transmettre des virus d'une culture à une autre, et nuire aux cultures de pleurotes.

Le but essentiel de la présente invention est de proposer de nouveaux pleurotes ne présentant pas les inconvénients liés à la présence de spores très nombreuses.

Un autre but de l'invention est de procurer des pleurotes particulièrement intéressants sur le plan économique en raison de leur rendement.

L'invention a pour objet, en tant que produits nouveaux, des pleurotes caractérisés en ce qu'ils présentent :

- le caractère asporulé,
- des lamelles décurrentes sur la majeure partie de la hauteur du pied,
- des lamelles plus développées que celles des autres souches de pleurotes.

Selon d'autres caractéristiques de l'invention :

- ils portent en général moins d'une dizaine de basides
  sporées par lamelle,
- leurs spores peuvent germer et le mycélium homocaryotique
  issu des spores porte le gène "sans spores".

L'invention a également pour objet :

- une souche de pleurotes asporulés, identifiée comme la souche SOMYCEL 3.200, caractérisée en ce qu'elle résulte du
  croisement des homocaryons PZ 21.3 et PZ 15 a1.
- un pleurote de la souche SOMYCEL 3.200 caractérisé en ce
  que son carpophore présente un pied dressé massif, et un
  chapeau bien développé, charnu et lourd,
- une souche de pleurotes asporulés, identifiée comme la
  souche SOMYCEL 3.210, caractérisée en ce qu'elle résulte du
  croisement des homocaryons PZ 25 a6 et PZ 15 a1;
- un pleurote de la souche SOMYCEL 3.210 caractérisé en ce
  que son carpophore présente un pied fin très long ; un
  chapeau petit, peu charnu et léger, à symétrie axiale et en
  forme de cornet ; et un défaut de géo-tropisme.

Les nouveaux pleurotes asporulés selon l'invention qu'ils
soient issus de la souche SOMYCEL 3.200 ou de la souche
SOMYCEL 3.210, présentent un certain nombre de caractéristiques communes et quelques caractéristiques particulières.

1. Caractéristiques physiologiques

1.1 - Caractéristiques communes.

Contrairement aux autres pleurotes connus, les pleurotes selon l'invention présentent le caractère asporulé, c'est-à-
dire qu'ils présentent peu de spores : en général moins d'une
dizaine de basides sporées par lamelle. Ces spores peuvent
germer, et le mycélium homocaryotique issu des spores porte
le gène "sans spores". Leur besoin de lumière est supérieur à
celui des souches connues, en intensité plutôt qu'en durée
d'éclairement. Ils fructifient dans une gamme de température
comprise entre 5 et 20°C.

## 1.2 - Caractéristiques particulières.

Pour la souche SOMYCEL 3.200, l'optimum de fructification se situe entre 12 et 18°C. A 12°C, le rendement sur un cycle normal de production est supérieur d'environ 40% à celui de la souche connue SOMYCEL 3.004.

Pour la souche SOMYCEL 3.210, l'optimum de fructification se situe entre 10 et 15°C. A 12°C, le rendement sur un cycle normal de production est supérieur d'environ 50% à celui de la souche SOMYCEL 3.004.

On peut donc constater un rendement nettement supérieur à la moyenne par rapport à la souche SOMYCEL 3.004.

## 2. Caractéristiques morphologiques

### 2.1 - Caractéristiques communes

Les pleurotes selon l'invention sont surtout remarquables par leurs lamelles qui sont très larges.

Les lamelles sont décurrentes sur la majeure partie de la hauteur du pied. Le pied présente un tissu lâche et lacunaire.

### 2.2 - Caractéristiques particulières

Pour la souche SOMYCEL 3.200, le chapeau est en général excentré par rapport au pied, mais il peut prendre une forme en cornet avec symétrie axiale, ou une forme enroulée. Il est bien développé, charnu et lourd. Le pied est dressé et massif, les lamelles sont décurrentes pratiquement jusqu'à la base du pied, la teinte générale varie du gris clair au marron.

Pour la souche SOMYCEL 3.210, le chapeau présente une forme en cornet, ou en trompette, avec symétrie axiale. Il est petit, peu charnu et léger. Le pied est fin et très long. Les lamelles sont décurrentes sur environ les deux tiers de la hauteur du pied. La teinte générale est grise. Ce pleurote est en

0161123

outre caractérisé par un défaut de géo-tropisme, et par le fait qu'il retombe vers le sol.

## 3. Caractéristiques génétiques

L'origine génétique des deux souches est commune : on part du néohaplonte 42 portant le caractère "sans spores", obtenu par dédicaryotisation selon le brevet FR-A-2.420.914. On procède à un croisement du néohaplonte 42 avec la souche SOMYCEL 3.004 et on sélectionne à partir de ce croisement les homocaryons portant le caractère "sans spores", identifiés comme homocaryons Z EGER. On procède à un croisement entre ces homocaryons "sans spores" Z EGER et la souche SOMYCEL 3.030. A partir de ce croisement, on sélectionne les homocaryons portant le caractère "sans spores", identifiés comme homocaryons PZ. On croise entre eux les homocaryons PZ 21.3 et PZ 15 a1 pour obtenir la souche SOMYCEL 3.200, et les homocaryons PZ 25 a6 et PZ 15 a1 pour obtenir la souche SOMYCEL 3.210.

Il faut remarquer que les homocaryons ne complémentent pas avec les testeurs "sans spores" 42 et 11 mentionnés dans le brevet précité FR-A-2.420.914.

Les facteurs d'incompatibilité sont : a3 b3, ayant pour origine la souche 42, et a1 b7, ayant respectivement pour origine la souche SOMYCEL 3.004 pour a1 et la souche SOMYCEL 3.030 pour b7.

Enfin, par rétrocroisement avec la souche SOMYCEL 3.030, les homocaryons redonnent le type sauvage 3.030 pour la couleur et la forme du champignon.

Par dédicaryotisation des souches SOMYCEL 3.200 et SOMYCEL 3.210, on obtient les néohaplontes présentant les caractéristiques précédentes.

On peut remarquer que les nouveaux pleurotes selon l'invention

0161123

présentent un certain nombre de caractères nouveaux et notamment : le caractère asporulé, des lamelles décurrentes sur la majeure partie de la hauteur du pied, et des lamelles plus développées que celles des autres souches de pleurotes.

Les pleurotes de la souche SOMYCEL 3.210 présentent en outre une forme en trompette et un défaut de géotropisme.

0161123

Revendications

1. - Pleurotes caractérisés en ce qu'ils présentent :

- le caractère asporulé,
- des lamelles décurrentes sur la majeure partie de la hauteur du pied,
- des lamelles plus développées que celles des autres souches de pleurotes.

2. - Pleurotes selon la revendication 1, caractérisés en ce qu'ils portent en général moins d'une dizaine de basides sporées par lamelle.

3. - Pleurotes selon la revendication 2, caractérisés en ce que leurs spores peuvent germer et le mycélium homocaryotique issu des spores porte le gène "sans spores".

4. - Souche de pleurotes selon la revendication 1, identifiée comme la souche SOMYCEL 3.200, caractérisée en ce qu'elle résulte du croisement des homocaryons PZ 21.3 et PZ 15 a1.

5. - Pleurote de la souche selon la revendication 4, caractérisé en ce que son carpophore présente un pied dressé massif, et un chapeau bien développé, charnu et lourd.

6. - Souche de pleurotes selon la revendication 1, identifiée comme la souche SOMYCEL 3.210, caractérisée en ce qu'elle résulte du croisement des homocaryons PZ 25 a6 et PZ 15 a1.

7. - Pleurote de la souche selon la revendication 6, caractérisé en ce que son carpophore présente un pied fin très long ; un chapeau petit, peu charnu et léger, à symétrie axiale et en forme de cornet ; et un défaut de géotropisme.

Office européen
des brevets

**RAPPORT DE RECHERCHE EUROPEENNE**

**0161123**
Numéro de la demande

EP  85  40  0447

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | DE-A-2 924 344  (SOMYCEL)<br>* Revendication 1 *<br><br>- - - - - | 1 | A 01 H  15/00 |

DOMAINES TECHNIQUES
RECHERCHES (Int. Cl.4)

A 01 G
A 01 H

Le présent rapport de recherche a été établi pour toutes les revendications

| Lieu de la recherche<br>LA HAYE | Date d'achèvement de la recherche<br>27-06-1985 | Examinateur<br>HERYGERS J.J. |
|---|---|---|